# EUROPEAN PATENT SPECIFICATION

(11) **EP 1 893 211 B1**
(45) Date of publication and mention of the grant of the patent: **14.09.2011**
(21) Application number: 06784962.0
(22) Date of filing: 15.06.2006
(51) Int. Cl.: A61K 31/5025, A61P 31/12, A61P 35/00, A61P 1/16

(54) **USE OF SANGLIFEHRIN IN HCV**
VERWENDUNG VON SANGLIFEHRIN BEI DER HCV-THERAPIE
UTILISATION DE SANGLIFÉHRINE DANS LE VIRUS DE L'HÉPATITE C

(30) Priority: 17.06.2005 US 691497 P
(43) Date of publication of application: 05.03.2008
(73) Proprietor: Novartis AG, 4056 Basel (CH); Novartis Pharma GmbH, 1230 Wien (AT)
(72) Inventor: LIN, Kai, Waltham, MA 02451 (US); WEIDMANN, Beat, Winchester, MA 01890 (US); ZIMMERMANN, Kaspar, CH-4104 Oberwil BL (CH)
(74) Representative: Vögeli-Lange, Regina
(86) International application number: PCT/US2006/023394
(87) International publication number: WO 2006/138507

(56) References cited:
- WO-A-97/02285
- WO-A-99/62540
- WO-A-2005/021028
- WO-A-2006/038088
- WO-A-2006/071619
- INOUE KAZUAKI ET AL: "Combined interferon alpha2b and cyclosporin A in the treatment of chronic hepatitis C: controlled trial." JOURNAL OF GASTROENTEROLOGY. 2003, vol. 38, no. 6, 2003, pages 567-572, XP002403244 ISSN: 0944-1174
- GOTO ET AL: "Evaluation of the anti-hepatitis C virus effects of cyclophilin inhibitors, cyclosporin A, and NIM811" BIOCHEMICAL AND BIOPHYSICAL RESEARCH COMMUNICATIONS, ACADEMIC PRESS INC. ORLANDO, FL, US, vol. 343, no. 3, 12 May 2006 (2006-05-12), pages 879-884, XP005358746 ISSN: 0006-291X
- SANGLIER, JEAN-JACQUES ET AL: "Sanglifehrins A, B, C and D, novel cyclophilin-binding compounds isolated from Streptomyces sp. A92-308110: I. Taxonomy, fermentation, isolation and biological activity" JOURNAL OF ANTIBIOTICS , 52(5), 466-473 CODEN: JANTAJ; ISSN: 0021-8820, 1999, XP008070099

## Description

The present invention relates to a new use for macrolide compounds of formula XVI of the sanglifehrin class that bind to cyclophilins.

The sanglifehrins are a class of polycyclic macrolide compounds initially isolated from actinomycete fermentation broths. Sanglifehrins have been found to exhibit activities to bind cyclophilins, and in the case of sanglifehrins A-D, immunosuppressive activities and inhibitory activities of both B-cell and T-cell proliferation. However, unlike the known immunosuppressant and anti-inflammatory compounds such as cyclosporine A and FK506, sanglifehrins do not have FKBP-binding activity or calcineurin inhibitory activity. Thus, sanglifehrins provide a novel category of drug substance both in terms of chemical structure and different activity profiles.

The sanglifehrins and the methods for their preparation are described in WO 97/02285, WO 98/07743, US 006124453; and J.Antibiotics 1999, 52(5), 466-473. A series of sanglifehrin derivatives, mini-sanglifehrins, are described in J.Am.Chem.Soc 2003, 125 (13), 3849-3859; J.Org.Chem. 2000, 65, 9255-9260; and Anger Chem. Int. Ed. 1999, 38(16), 2443-2446. The use of cyclosporins and modified cyclosporins are described in WO99/62540 and WO2005/021028 respectively. The use of a combination of interferon alpha-2b and cyclosporin A in the treatment of chronic hepatitis C is described in J. Gastroenterology 2003, 38(6), 567-572.

It has now surprisingly been found that the cyclophilin-binding compounds of formula XVI are useful for the treatment of hepatitis C, liver fibrosis, liver cirrhosis and hepatocellular carcinoma.

Sanglifehrins are isolated from actinomycete fermentation broths. Commonly known sanglifehrins include sanglifehrin A through D, e.g. of the formula

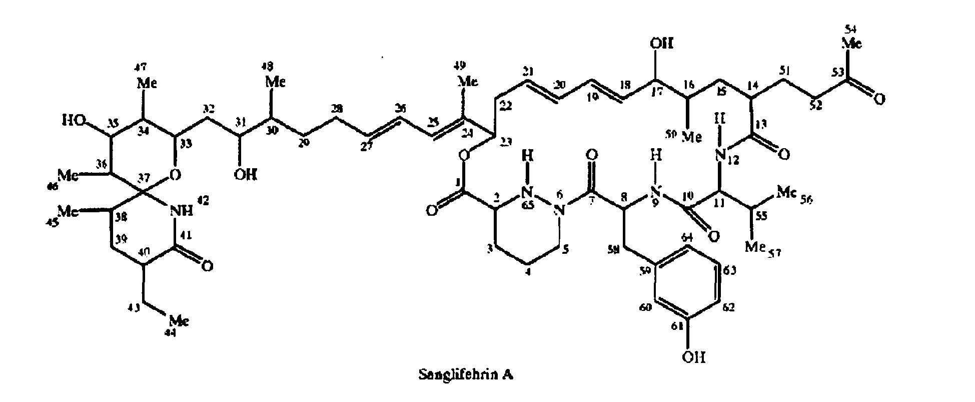

The macrocyclic ring of Sanglifehrins A to D is characterised in that i) positions 1 to 6 comprise a 3- carboxypiperidazinyl carboxylic acid residue, ii) positions 7 to 9 an aromatic alpha-amino acid residue, and iii) positions 10 to 12 an aliphatic alpha-amino acid residue. The remainder of the macrocyclic ring is comprised by an hydroxy carboxylic acid residue, providing, in the case of Sanglifehrins A to D a further 11 carbon atoms in the primary macrocyclic ring.

In accordance with conventional practice in macrolide chemistry the atoms of the Sanglifehrin primary macrocyclic ring are numbered as indicated above for Sanglifehrin A, starting with the carbon atom of the carbonyl group of the macrocylic lactone linkage as position 1.

Sanglifehrins A to D may also be characterised by the presence of a novel bicyclic Spiro system attached at the 23 position of the macrocyclic ring via a hydrocarbyl linker group.

Sanglifehrins A to D may be subjected to extensive chemical manipulation to obtain yet further macrolides of the Sanglifehrin class. Such manipulations include cleavage of the macrocyclic ring, in particular at the lactone oxy-group, cleavage of the linker group between the macrolide and Spiro ring systems, and manipulation, e.g. protection, derivatization or other chemical modification of substituent groupings; for example, as hereinafter described. Further means of modification will be apparent to those skilled in the art.

As described herein Sanglifehrins have a characteristic and entirely novel profile in terms of their biological activity against hepatitis C infections. In particular they exhibit the combination of activities as hereinafter described.

Sanglifehrins have an activity profile that differs from that of previously known immunosuppressant and antiinflammatory compounds such as cyclosporins and macrolides, e.g. of the rapamycin and NIM811, indicating that the Sanglifehrins have a different mode of action than such compounds. Thus the Sanglifehrins provide a novel category of drug substance both in terms of structure and activity which may be anticipated to materially extend the bounds of immunosuppressive, antiinflammatory or antiviral therapy; for example, to avoid or reduce undesirable side effects of previous immunosuppressive and antiinflammatory therapies and/or to improve or extend such therapy to new disease areas or new patient categories.

Sanglifehrins, e.g. in which the macrolide ring is in ring-opened form, in which the 26 and 27 positions in the hydrocarbyl linker between the macrolide and spiro ring systems are both hydroxy substituted, or in which the spiro residue attached to the macrocyclic ring has been cleaved or truncated, generally lack some or all of the combination of Sanglifehrin characteristic activities. For example, Sanglifehrins in which the spiro residue is cleaved typically possess cyclophilin binding activity but do not possess significant immunosuppressive activity. As will be apparent to those skilled in the art, however, such compounds provide valuable components, intermediates or key building blocks for the preparation of further novel Sanglifehrins, and hence further extend the therapeutic potential of the Sanglifehrin class.

In that its presence appears material to the biological activity, e.g. of the Sanglifehrins A to D, the bicyclic spiro system may also be viewed as providing a structural component with key biological significance, useful as a structural component for further derivatization or modification both in relation to the production of further Sanglifehrins or for application in the derivatisation or modification of other drug substances for example, to modify the activity of other immunosuppressive drug substances of the macrolide class.

Herein described is a macrolide in which i) positions 2 to 6 inclusive of the macrocyclic ring are provided by a piperidazinyl carboxylic acid residue; and/or ii) positions 7 to 9 inclusive of the macrocyclic ring are provided by an aromatic .alpha.-amino acid residue; and/or iii) positions 10 to 12 inclusive of the macrocyclic ring are provided by an aliphatic .alpha.-amino acid residue, in free or protected form, or a salt thereof.

Suitably the macrolides described herein may comprise two, especially all three of the characteristic structural features i), ii) and iii).

The piperidazinyl carboxylic acid residue described herein is suitably a 1,2-piperidazin-3-carboxy-1-yl residue of which the carboxy moiety occupies the 1-position, and the 1-nitrogen atom the 6-position of the macrocyclic ring, e.g. a residue of formula I 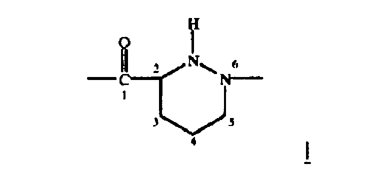 wherein the assigned numbers represent the position of the atoms of the residue in the macrocyclic ring. This residue may be ring substituted or unsubstituted. Suitably it is unsubstituted.

The carboxy moiety of the aromatic alpha-amino acid residue described herein suitably occupies the 7-position of the macrocyclic ring, and the alpha-amino moiety the 9-position. Suitably the aromatic aipha-amino acid described herein is a substituted or unsubstituted phenylalanine, especially by hydroxy, methoxy, ethoxy, OnPr, OiPr, residues in free, protected or activated form.

The carboxy moiety of the aliphatic alpha-amino acid residue described herein suitably occupies the 10-position of the macrocyclic ring, and the alpha-amino moiety the 12-position. Suitably the aliphatic alpha- amino acid residue described herein is a valine residue in free, protected or activated form.

The remainder of the herein described macrocyclic ring suitably comprises a hydroxy carboxylic acid residue, the oxy-moiety of which completes the macrocyclic lactone linkage and the carbonyl moiety of which forms an amide linkage with the alpha-amino group at position 12 of the macrocyclic ring. The said hydroxy carboxylic acid residue described herein suitably has a chain length of from 6 to 20, more suitably 11 carbon atoms. It may be substituted or unsubstituted and/or contain one or more unsaturated linkages in particular cumulative double bonds along its length. More suitably the remainder of the macrocyclic ring described herein comprises a 11-oxy-undecanoyl-11-yl, especially 11-oxy-undeca-6,8-dienoyl-11-yl, residue optionally substituted, e.g. in the 2, 3, 4 and/or 5 positions. More suitably the said hydroxy carboxylic acid residue described herein is a residue of formula II 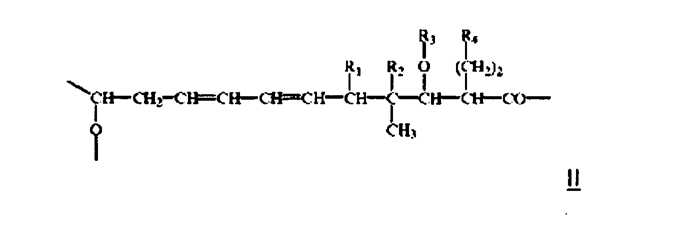 wherein R1 is H, OH or represents an extra bond and R2 is H or represents an extra bond; R3 is H, and R4 is -CO--CH₃ or --CH(OH)-CH₃ or R3 and R4 together represent a structure of formula III

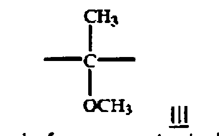

In free or protected form, or salt thereof.

Preferred macrolides herein described are those comprising a macrocylic ring of formula IV 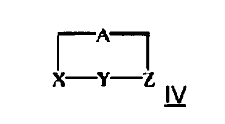 wherein X, Y and Z are residues i), ii) and iii) as defined above and A is a hydroxy carboxylic acid residue as defined above in free or protected form, or salt thereof; in particular a macrocyclic ring of formula V

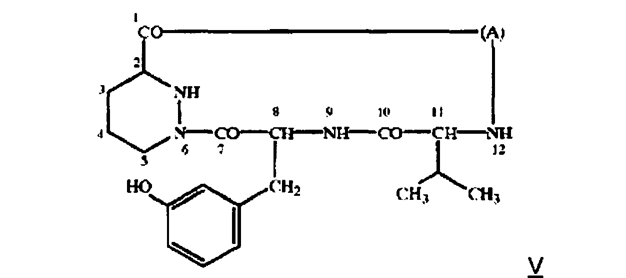

in free or protected form, or salt thereof

Generally in the Sanglifehrins, the macrocyclic ring is substituted at the carbon atom adjacent the oxy moiety of the lactone bridge. Typically this substituent comprises a 1-oxo-7-aza-spiro-{5. 5}-undecan-8-one-2-yl residue, e.g. of the formula VI 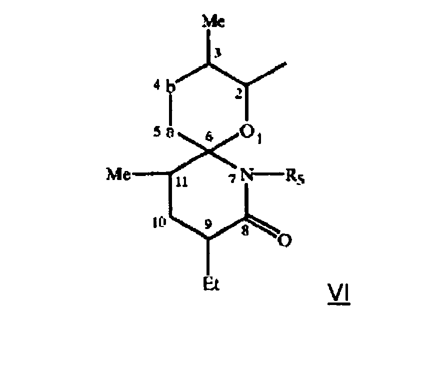 wherein --a--b-- is -(Me)C=CH-- or --(Me)CH--CH(OH)- and R, is H or Me (wherein Me and Et represent methyl and ethyl respectively) in free or protected form, or salt thereof linked to the macrolide ring via a linker comprising a linear sequence of from 6 to 11, typically 9, carbon atoms between the spiro residue and the macrolide ring.

The linker group may be substituted or unsubstituted and/or contain one or more unsaturated linkages in particular cumulative double bonds along its length. Suitably the linker group described herein may be methyl substituted, e.g. by two methyl groups. Suitably the linker group described herein may be further substituted by hydroxy, e.g. by three hydroxy substituents, and/or may be ethylenically unsaturated, e.g. contain two carbon-carbon double bonds. More suitably the linker group described herein comprises a 1,7-dimethyl- nonan-9-yl, especially a 1,7-dimethyl-non-1-en-9-yl or a 1,7-dimethyl- nona-1,3-dien-9-yl, residue optionally substituted, e.g. in the 3, 4, and/or 8 position. Preferably the linker group is a group of formula VII 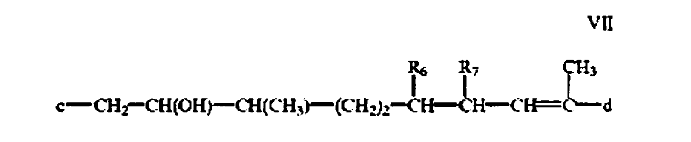 wherein c represents linkage to the spiro residue; d represents linkage to the macrocyclic ring and R6 and R7 are each OH or together represent an additional bond, in free or protected form.

The linker group described herein will generally be attached to the macrocyclic ring at the carbon atom immediately adjacent to the lactone oxy group, i. e. when the macrocyclic ring comprises an 11-oxy-undecanoyl-11-yl residue, at the 11 position of thereof.

Herein described are compounds of formula VIII

S--L-M VIII

wherein S represents a spiro bicyclo residue as previously defined; L represents a linker group as previously defined, and M represents a macrolide ring as previously defined, in free or protected form, or salt thereof.

Particular compounds described herein are those of formula IXa and IX 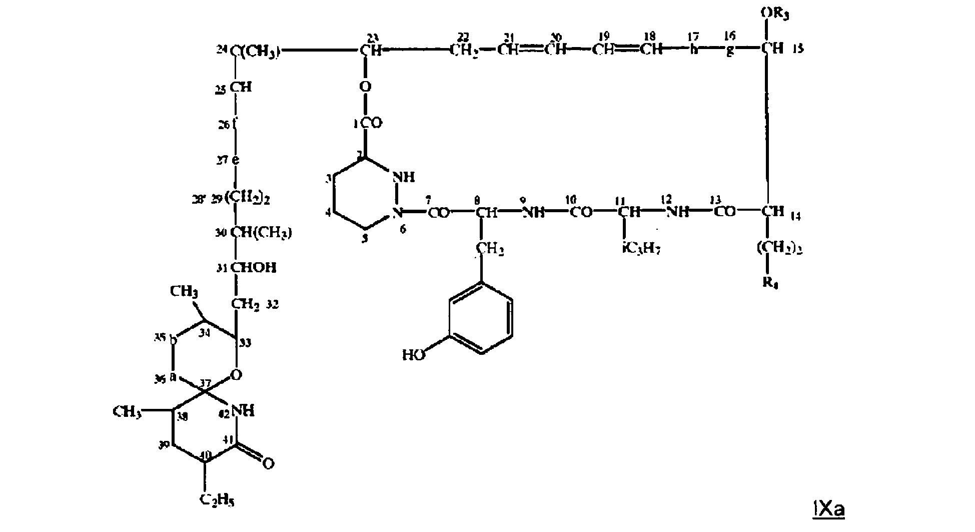 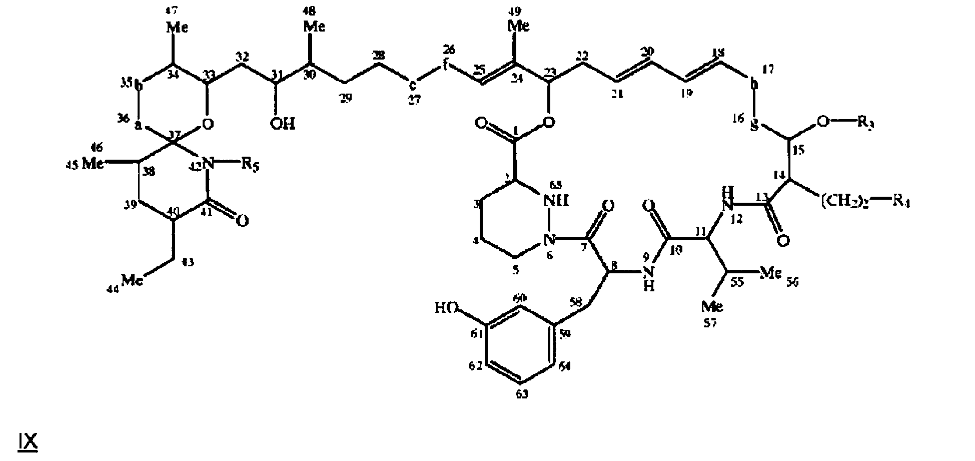 wherein --a--b- is as defined above, --e-f--is --CH(OH)--CH(OH)-- or -CH=CH--; --g--h- is as defined above for --a--b--, and R3, R4 and R5 are as defined above, in free or protected form or salt thereof

The compounds of formulae I to IX contain asymmetric carbon atoms and thus may exist in a number of epimeric forms. All of the possible epimers as well as diastereoisomeric mixtures thereof are described herein. However, compounds of formulae VIII and IX described herein in which the macrolide ring is in ring-closed form and which are of appropriate stereochemistry possess activities which are characteristics of Sanglifehrins, as herein referred to. Epimers described herein which possess sanglifehrin characteristic activities are preferred. In general, epimers which possess sanglifehrin characteristic activities in pure or substantially pure form (i.e. free or substantially free of epimers which lack sanglifehrin characteristic activities), e.g. comprising at least 90%, e. g. at least 95% of active epimer (i.e. comprising less than 10%, e.g. less than 5% of inactive epimer) will be preferred.

Preferably the 3-carboxypiperidazinyl carboxylic acid residue i) at positions 1 to 6 of the macrocyclic ring described herein has the following conformation:

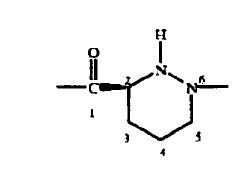

Preferably the aromatic amino acid ii) at positions 7 to 9 of the macrocyclic ring described herein has the L configuration, e.g. is of configuration

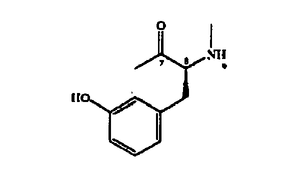

Preferably the aliphatic amino acid iii) at positions 10 to 12 of the macrocyclic ring described herein has the L configuration, e.g. is of configuration

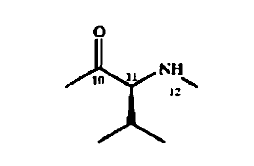

When the remainder of the macrocyclic ring described herein comprises a residue of formula II, it preferably has the configuration 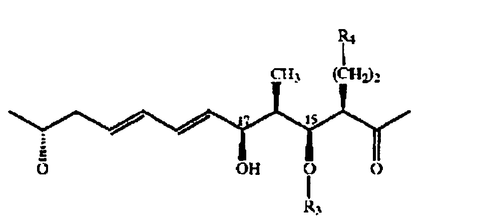 or

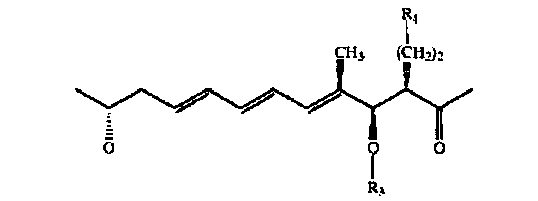

When R3 and R4 together preferably are of the configuration

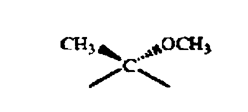

Preferably the 1-oxo-7-aza-spiro-{5.5}-undecan-8-one-2-yl residue described herein has the configuration 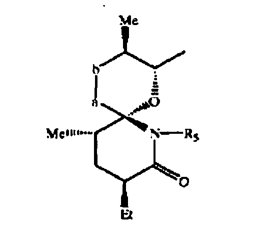 wherein when --a--b-- is --(Me)CH--CH(OH)-, it preferably has the configuration

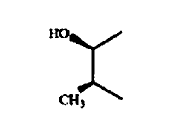

When the linker described herein is of formula VII, it is preferably of configuration

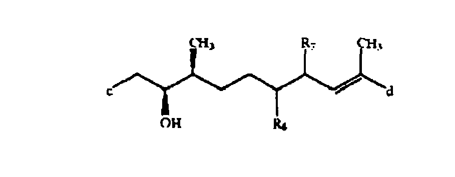

When R6 and R7 are each OH, the configuration at positions 26 and 27 is preferably either 26(S), 27(S) or 26(R), 27(R). When R6 and R7 together represent an additional bond, the configuration at positions 26 and 27 is preferably

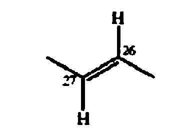

Compounds of formula IX described herein preferably have the following conformation 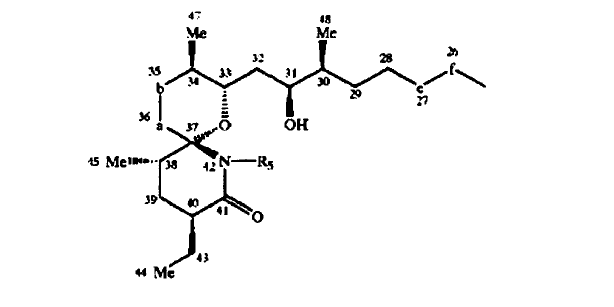 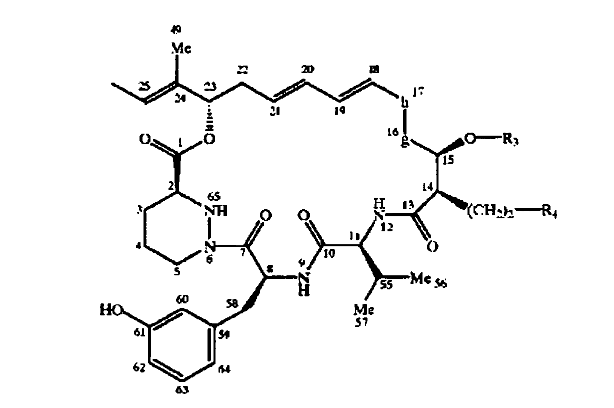 wherein when --a-b-- is --(Me)CH--CH(OH)-, it preferably has the configuration: 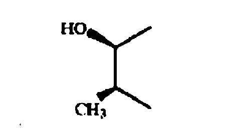 when --e--f-- is --CH(OH)--CH(OH)-, it preferably has the (S),(S) configuration or the (R),(R) configuration; when -g--h-- is --(Me) CH--CH(OH)-, it preferably has the configuration: 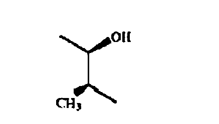 when --g--h-- is -- (Me)C=CH-, it preferably has the configuration: 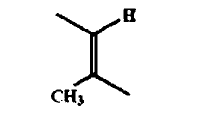 and when R3 and R4 are fused together they are preferably of configuration

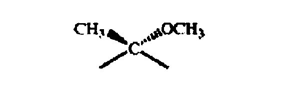

The compounds described herein may be in free or protected form, e.g. in protected forms as described in "Protective Groups in Organic Synthesis" by T. W. Greene and P. G. M. Wuts, 2nd Edition, 1991, John Wiley & Sons Inc., New York. In particular OH groups may be in protected form, e.g. in the form of silyl ethers (for instance as described in pages 68-86 of Greene and Wuts ibid.), esters (see e.g. pages 87-103 of Greene and Wuts ibid) and carbonates (see e. g. pages 104- 111 of Greene and Wuts ibid). Such protected forms also include internally protected forms; for example, in the case of macrolides of formula IX, wherein --g--h-- is --CH(CH₃)--CH(OH)--, the protected form wherein the 14 to 17 positions of the 15 macrocyclic ring comprise a residue of formula X:

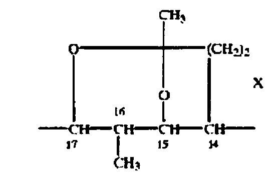

Also for example, 1,3 diols present in Sanglifehrins may be protected as appropriate ring structures, e.g. as described on pages 118-142 of Greene and Wuts ibid.

Compounds described herein also exist in salt form. Examples of suitable pharmaceutically acceptable salts include acid and base addition salts as appropriate having, regard to the particular substituents present in the compound, e.g. HCl salt forms.

As previously indicated the macrocyclic ring of the compounds described herein can be cleaved, in particular at the lactone oxy group, to provide compounds wherein the macrocyclic ring is in ring-open form. Generally cleavage of the lactone oxy group proceeds by hydrolysis (solvolysis), e.g. to provide ring-open macrocycle compounds of formula XI:

R6 O-X-Y--Z-A--OH XI

e.g. of formula XII 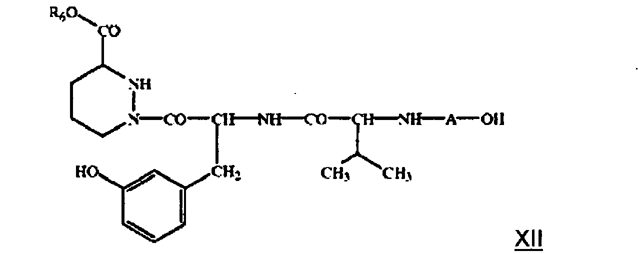 e.g. a compound of formula IX' 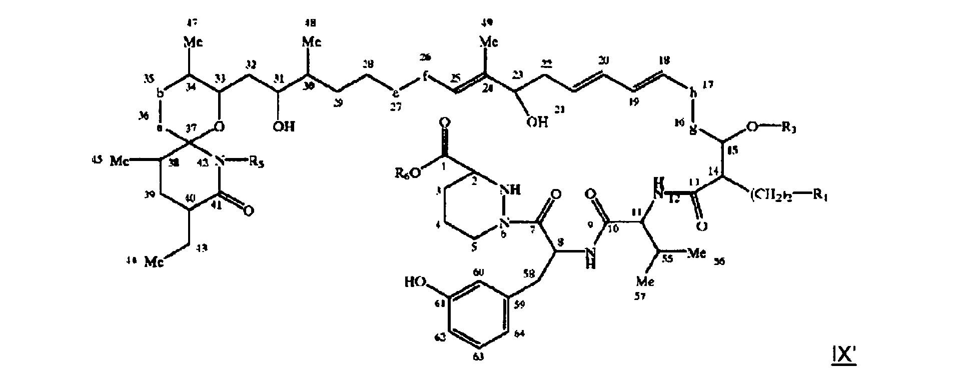 wherein X, Y, Z, A, --a--b-, --e-f--, --g--h-, R3, R4 and R5 are as defined above and R6 is H or C₁₋₄ alkyl, e.g. methyl.

Such ring-opened forms provide intermediate means for modification of the basic Sanglifehrin macrocyclic ring system and are also described herein.

Herein described is a macrolide as hereinbefore defined in ring-opened form, said ring-opened macrocycle being in free or protected form, or salt thereof; a compound R6 O--X-Y--ZA--OH as defined above, in free or protected form, or salt thereof; a compound R6 O--X-Y--Z-A'--CH(OH)--L--S, wherein --A'-- CH(OH)-- is a hydroxy carboxylic acid residue, e.g. a residue of formula II, as defined above, and the other symbols are as defined above, in free or protected form, or a salt thereof;
a compound of formula XII' 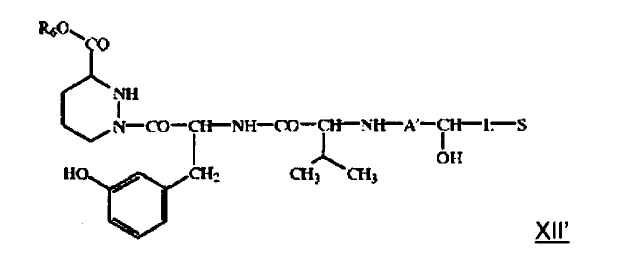 in free or protected form, or salt thereof;
a compound of formula IX' 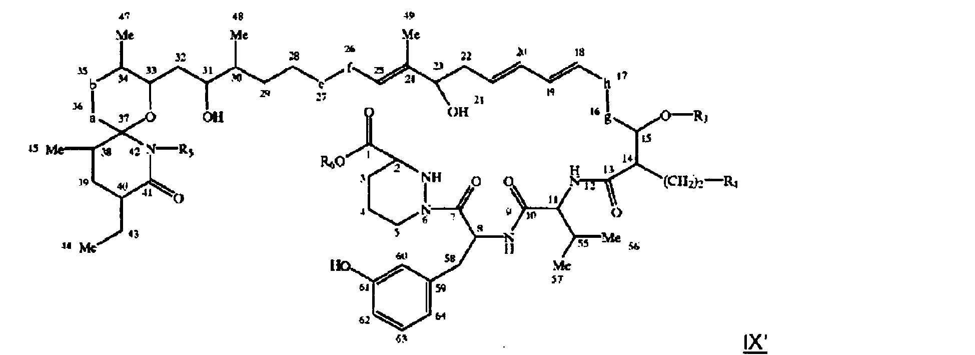 in free or protected form or a salt thereof.

Herein described is a compound of formula IX" 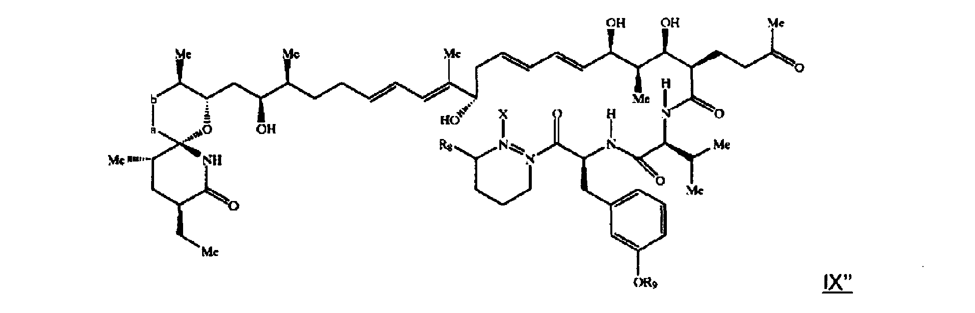 wherein --a--b-- is --(Me)C=CH- or -(Me) CH--CH(OH)--; R8 is H or --C(O)--OR10, wherein R10 is H or Me; R9 is H or, when --a--b-- is --(Me)CH--CH(OH)-- and R is -- C(O)-OMe, R9 is Me, and X is H when --N----N-- represents a single bond between the two N atoms, or X together with --N---N- represents a double bond between the two N atoms, in free or protected form or a salt thereof.

Herein described are compounds of formulae W, Y (Seco-sanglifehrin A), S (Seco-sanglifehrin B) and T

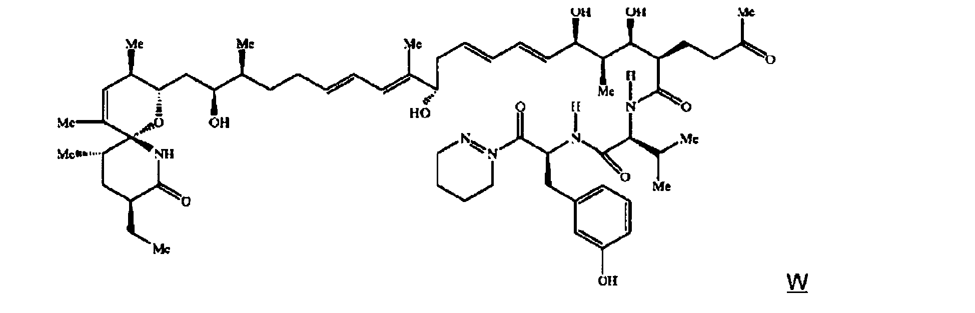

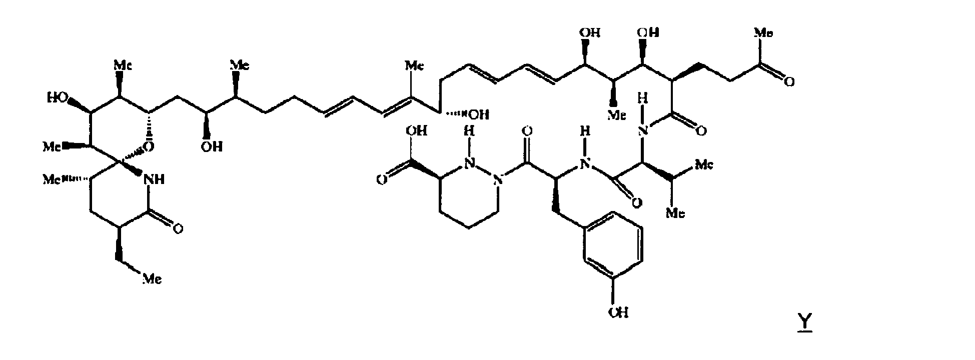

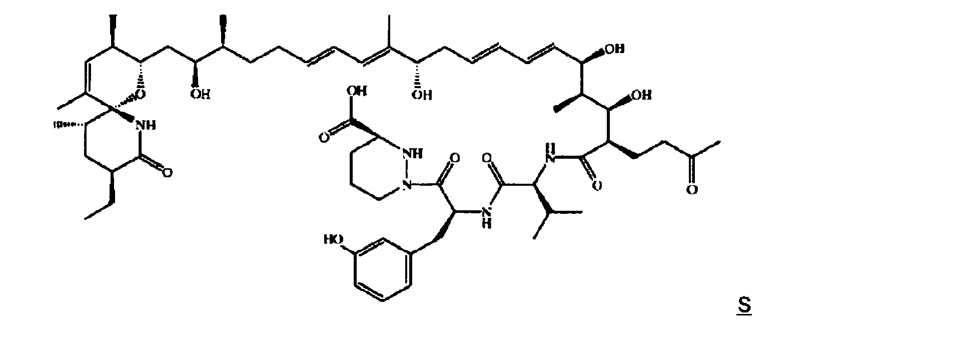

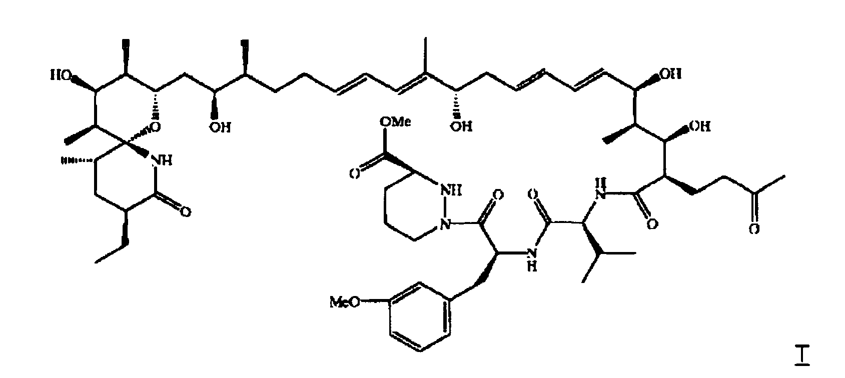

Also herein described are includes compounds in which the 1-oxo-7-aza-spiro-{5,5}-undecan-8-one-2-yl ring system is in ring-opened form, e.g. a compound of formula XIII 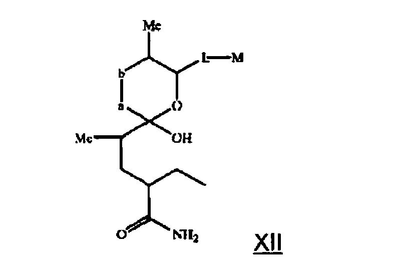 wherein a, b, L and M are as defined above, in free or protected form, or a salt thereof.

The ring-opened compounds described herein are preferably of conformation as the preferred conformations identified above for ring- closed compounds. The ring-opened spiro bicyclo ring system of compounds of formula XIII is preferably of conformation: 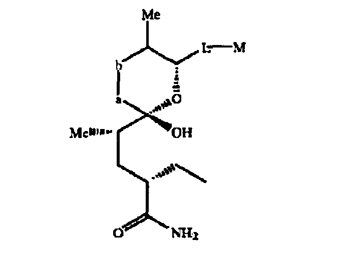 wherein when --a-b- is --(Me) CH--CH(OH)-,

Herein described is a compound of formula Z (Sanglifehrin E) 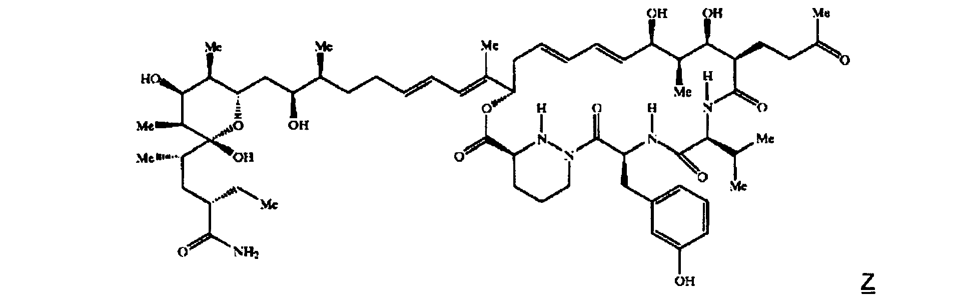 in free or protected form, or a salt thereof.

The compounds of formulae IX", S, T, W, Y, XIII and Z described herein are useful intermediates for the preparation of sanglifehrins in general including ring closed forms and ring expanded ring closed forms.

The compounds of formulae IX" described herein may be prepared by cleavage of the macrolide ring of a ring closed sanglifehrin, e.g. sanglifehrin A or B, at or in the vicinity of the lactone oxy group. Thus, for example, the compounds of formula Y and T are prepared by treatment of sanglifehrin A under basic conditions, e.g. for the compound of formula III in the presence of an alkali metal hydroxide and e.g. for the compound of formula IV in the presence of methanol and an alkali metal carbonate.

Alternatively compounds of formula IX" described herein may be obtained as isolates from cultures of a sanglifehrin producing microorganism. For example, the compounds of formula Y and S may be obtained as isolates from the cultures of Streptomyces sp. A92-308110 as hereinafter described.

The isolation of the compounds of formulae Y and S from cultures of Streptomyces sp. A92-348110 is described in US6124453.

The compound of formula Z may be obtained by cleavage of the spirobicyclic ring system between the nitrogen atom and the central atom of the spiro system. Alternatively the compound of formula Z may be obtained as an isolate from a culture of a sanglifehrin producing microorganism. For example, the isolation of the compound of formula Z from cultures of Streptomyces sp. A92-308110 is described in US6124453.

Macrolides described herein having a spiro bicyclo residue attached to the macrocyclic ring may also be subjected to cleavage of the intervening linker group, e.g. in relation to formula IX, in particular at the linkage between residues 26 and 27 to yield separate novel Spirobicyclo compounds and further macrolides. As also previously indicated these compounds are useful as intermediates, the spiro bicyclic moiety of the Sanglifehrins in particular having an integral functional role in the biological activity of the Sanglifehrins as a class.

Herein described is a 1-oxo-7-aza-spiro-{5.5}-undecan-8-one-2-yl, in free or protected form, or a salt thereof, in particular a compound of formula VI' 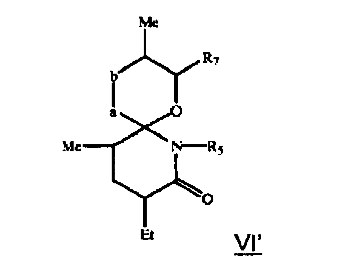 wherein R7 is H, an optionally protected OH group, a reactive functional group, or a-CH₂ -CH(OH)-- CH(CH₃)--CH₂ -CH₂ --CHO group or the delta lactol equivalent thereof, in free or protected form or salt thereof.

Preferably the compound of formula VI' described herein has the following configuration 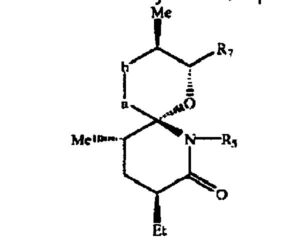 wherein when --a--b-- is --(Me)CH--CH(OH)-, it preferably has the configuration as previously defined.

Herein described isalso a ring-open 1-oxo-7-aza-spiro-{5.5}-undecan-8-one-2-yl, in free or protected form, or a salt thereof, in particular a compound of formula XIII' 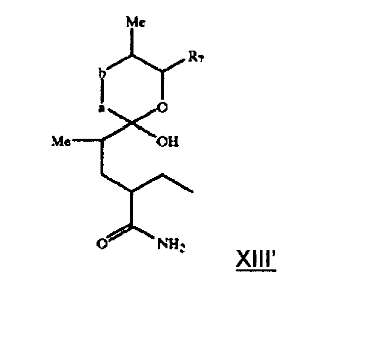 wherein --a--b-- and R7 are as previously defined, in free or protected form, or a salt thereof. The ring- opened spiro bicyclo ring system of compounds of formula XIII' described herein is preferably of conformation: 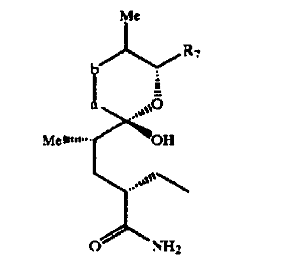 wherein when -a-- b- is --(Me)CH--(OH)-, it preferably has the configuration as previously defined.:

Herein described isalso a macrolide of formula XIV 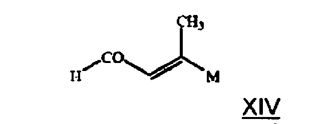 wherein M is a macrolide ring as defined above, in particular a macrolide of formula XV 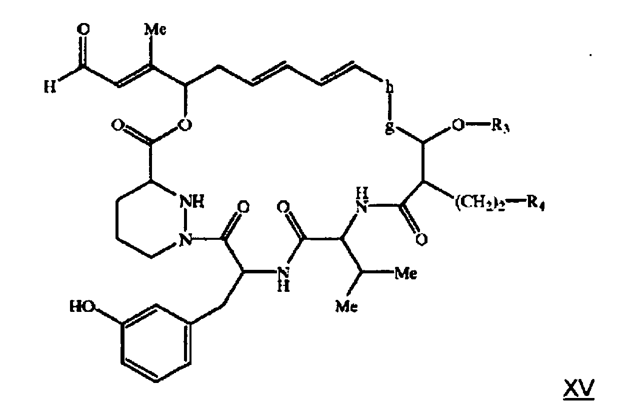 preferably of conformation 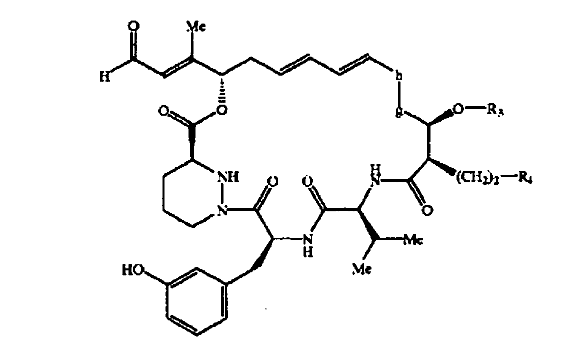 wherein when -g-h-- is --(Me) CH--CH(OH)-, it preferably has the configuration as previously defined and when --g--h-- is -(Me)C=CH--, it preferably has the configuration as previously defined and when R3 and R4 are fused together they are preferably of configuration as defined herein, in free or protected or ring-opened form, or a salt thereof.

The invention provides a macrolide of formula XVI 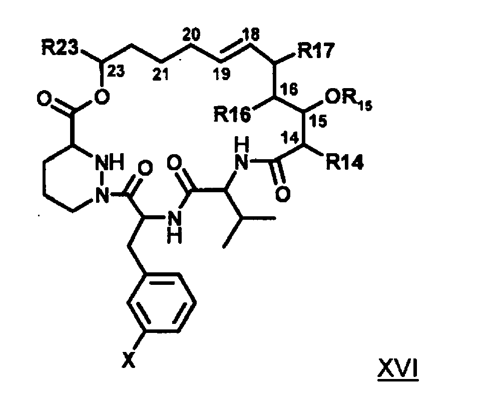 wherein X is H, methoxy, ethoxy, OnPr, OiPr;
R14 and R15 are independently H, methyl, alkyl, (C₁-C₄)alkyl, (C₁-C₄)-CO-CH₃, or R14 and R15 are fused to form a 5 to 7 membered substituted or unsubstituted cycloalkyl: selected of formula 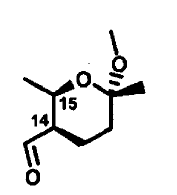 R16 is H, or Me or (C₁₋₄)alkyl;

R17 is H, OH, -O-(C₁₋₄)alkyl or R14, R15 and R17 are fused to form a cycloalkyl of formula

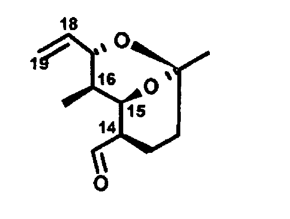

R23 is selected from the group consisting of -CH=CH₂,-CH₂OH, -CH₂-OCH₃, -CHO, - COOH, 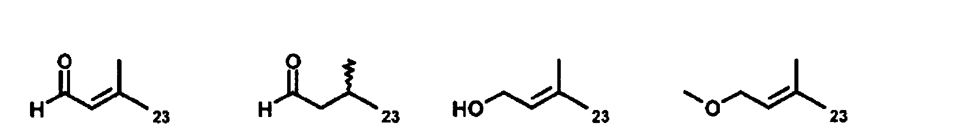 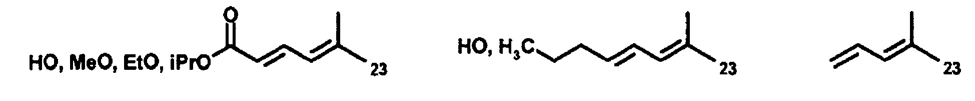 for use as indicated above.

Herein described are macrolides are selected from formula XX

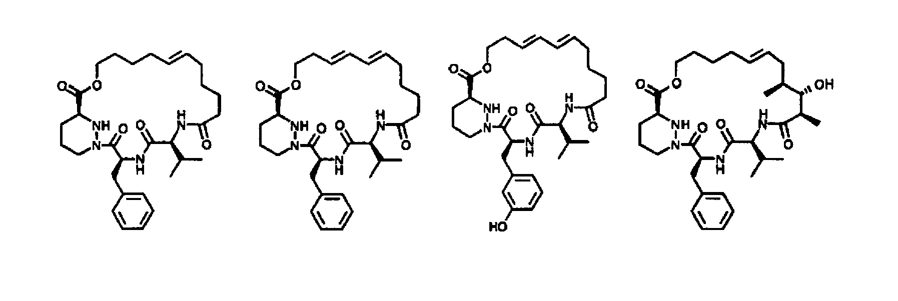

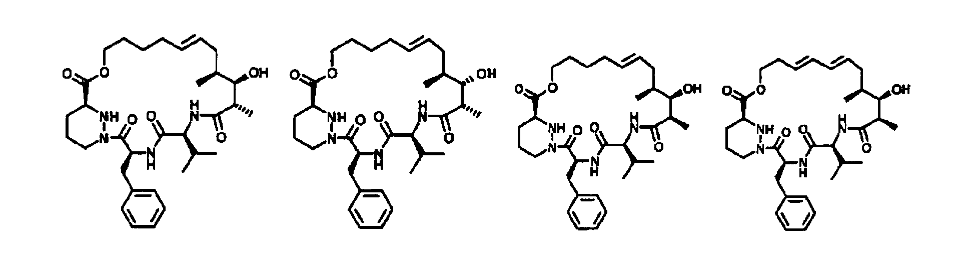

Sanglifehrins A, B, C, and D, amongst others, are isolated from the novel Streptomyces sp. A92- 308110. Samples of Streptomyces sp. A92-308110 are deposited with the Deutsche Sammlung von Mikroorganismen und Zelikulturen GmbH, Mascheroder Weg 1b, D-38124 Braunschweig, Germany on the May 3, 1995 under the terms of the Budapest Treaty and have been assigned deposition number DSM 9954. The isolation of Sanglifehrins A, B, C, and D from cultures of Streptomyces sp. A92-30810 is described in US6124453.

Macrolides described herein, e.g. compounds of formula IX; for example, Sanglifehrins A, B, C and D, and their pharmaceutically acceptable salts, , exhibit sanglifehrin characteristic activities, i.e. the following combination of activities:
have cyclophilin binding activity;
have inhibitory activity against peptidylprolyl cis-trans isomerase;
have immunosuppressive activity;
inhibit proliferation of both B-cells and T-cells;
but do not have FK binding protein binding activity, and
do not inhibit calcineurin activity.

Biological activity of macrolides of the formula XVI may be demonstrated in standard in vitro and in vivo test methods, e.g. as follows.

Proliferative Response Of Lymphocvtes to Allogenic Stimulation may be prepared as follows. Spleen cells (2x10⁵) from Balb/c mice (female, 8-10 weeks) are co-incubated for 4 days with 2x10⁵ spleen cells from CBA mice (female, 8-10 weeks). The allogenic cells induce a proliferative response in the responder spleen cell population which is measured by labelled precursor incorporation into the DNA. Macrolides of the inventionhave IC₅₀ in the range from about 30 up to about 200 nM as compared with an IC₅₀ of about 20 nM for cyclosporin A when tested in this assay. (T. Meo (1979) The MLR in the mouse. In: "Immunological Methods", L. Lefkovits and B. Pemis, Eds. Academic Press, N.Y. pp. 227-239)

Spleen cells (2x10⁵) from CBA mice are incubated for 48 hours with 50 .mu.g/ml LPS plus test compound. Proliferation is measured by labelled precursor incorporation into DNA. Macrolides of the invention, inhibit B-cell proliferation and have IC₅₀ in the range from about 40 up to about 100 micromolar. (Greaves, M. and J. Janossy, 1972, Elicitation of selective T and B lymphocyte response by cell surface binding ligands, Transplant Rev., 11:87 Janossy, G. and M. F. Greaves, 1971, Lymphocyte activation, I, Response of T and B lymphocytes to phytomitogens, Clin. Exp. Immunol. 9:483-498).

Cytotoxicity is determined using the human monocytic cell line THP1 (5x10⁴ cells/well) which are incubated in the presence of IFNgamma (100 U/ml) and LPS (5 microgram/ml) plus test compound (up to 10 micromolar) for 24 to 72 h at 37.degree. C. Living cells are quantified using the colourimetric read-out MTT which measures mitochondrial dehydrogenase enzymatic activity in living cells (Mossman 1983). Macrolides of the inventionhave IC₅₀ of about 1000-5000 nM after 24 h incubation in this assay. (Mossman T. J. (1983), Rapid calorimetric assay for cellular growth and survival: application to proliferation and cytotoxic assays, J. Imm. Methods, 85, 55-63).

Mononuclear cells are prepared from the peripheral blood of healthy volunteers using Ficoll-Hypaque density separation according to the method of Hansell et al. (1991). Cells (10⁵ cells/well in 200 microliters RPMI 10% FCS by volume) are incubated with serial dilutions of the test compounds for 30 min at 37.degree. C. prior to the addition of stimulus. Interferon gamma (100 U/ml) and LPS (5 microgram/ml) are used as stimuli to induce Tumour Necrosis Factor (TNF)alpha release by peripheral blood mononuclear cells. After 3 h incubation, the cells are centrifuged (1200 rpm for 10 min) and the supernatants are collected. The amount of TNF present in the cell supernatants is determined using a commercially available enzyme-linked immunosorbent assay kit. Macrolides of the invention have IC₅₀ in the range from about 200 nm to about 1000 nm when tested in this assay.

A suitable cyclophilin binding assay is the competitive ELISA test described by Quesniaux in Eur. J. Immunol. 1987 17 1359-1365. In this test, the compound to be tested is added during the incubation of cyclophilin (human recombinant cyclophilin A) with coated BSA-cyclosporin A and the concentration required to give a 50% inhibition of the control reaction without competitor is then calculated (IC₅₀). An alternative assay is the competitive binding test described by Schneider et al. in Biochemistry (1994), 33, 8218-8224, which involves addition of test compound during the incubation of biotinylated cyclophilin (human recombinant cyclophilin A) with coated BSA- cyclosporin A. The amount of biotinylated cyclophilin bound in the presence and absence of a test compound is determined by incubation with streptavidin-coupled alkaline phosphatase. Macrolides of the invention have IC₅₀ in the range from about 10 to about 100 nM, compared with an IC₅₀ of about 80 nM for cyclosporin A when tested in these assays.

Further in vitro assays which may be used to demonstrate the biological activity of Sanglifehms are IL-2 reporter gene assays and ConA-stimulated spleen cell assays (indicative of effect on T-cell activation).

Macrolides of the inventiondo not have FK binding protein binding activity and do not inhibit calcineurin activity when tested in standard tests for these activities.

Herein described are also
1.1 A method for preventing or treating hepatitis C infections or HCV induced disorders such as liver fibrosis, liver cirrhosis and hepatocellular carcinoma in a subject in need thereof, comprising administering to said subject a therapeutically effective amount of a cyclophilin-binding compound such as a sanglifehrin.
1.2 A method for inhibiting HCV replication in a medium, comprising applying to this medium an effective amount of a cyclophilin-binding compound such as a sanglifehrin.
1.3 A method for inhibiting HCV replication in a patient in need thereof, comprising administering to this subject a therapeutically effective amount of a cyclophilin-binding compound such as a sanglifehrin.
1.4 A method for preventing the recurrence of HCV infection in a transplant recipient in need thereof, comprising administering to said recipient a therapeutically effective amount of a cyclophilin-binding compound such as a sanglifehrin.
2. Use of a cyclophilin-binding compound such as a sanglifehrin, e.g. a compound of formula _, in the preparation of a pharmaceutical composition for use in any method as defined above.
3. A pharmaceutical composition for use in any method as defined above, comprising a cyclophilin-binding compound such as a sanglifehrin, e.g. a compound of formula _, together with one or more pharmaceutically acceptable diluents or carriers therefor.

In addition compounds of formula XVI of the invention which possess cyclophilin binding activity, may be useful as reagents in displacement immunoassays for cyclosporins and other cyclophilin binding compounds, for example in the assay procedure described patent application WO 95/07468. This patent application relates to an assay procedure for determining the concentration of an immunophilin-binding pharmaceutical, e.g. Cyclosporin, in blood; the procedure comprising adding a binding competitor that displaces the pharmaceutical from immunosuppressant- immunophilin complexes in the blood; adding a receptor that binds to the pharmaceutical but not significantly to the binding competitor; separating the receptor-pharmaceutical complex from the sample; and determining the amount of the pharmaceutical. compounds of formula XVI may be used as the binding competitor in such assays; for instance, to displace cyclosporins from cyclophilins, thereby releasing the cyclosporin for quantitation, e.g. by a monoclonal antibody which is specific for the cyclosporin.

In accordance with the foregoing herein described is:
4. A pharmaceutical combination comprising a) a first agent which is a cyclophilin-binding compound such as a sanglifehrin e.g. a compound of formula I, and b) a co-agent, e.g. one or more drug agents as provided herein.
5. A method as defined above comprising co-administration, e.g. concomitantly or in sequence, of a therapeutically effective amount of a cyclophilin-binding compound such as a sanglifehrin, e.g. a compound of formula I, and a co-agent, e.g. one or more drug agents as provided herein.

The compound of the invention may be administered by any conventional route, in particular enterally, e.g. orally, for example in the form of solutions for drinking, tablets or capsules or parenterally, for example in the form of injectable solutions or suspensions. Preferred pharmaceutical compositions may be e.g. those based on microemulsions as described in UK 2,222,770 A.

Co-agents can be co-administered in combination or alternation treatment. The terms "co-administration" or "combined administration" or the like as utilized herein are meant to encompass administration of the selected therapeutic agents to a patient, and are intended to include treatment regimens in which the agents are not necessarily administered by the same route of administration or at the same time. Fixed combinations are also describedherein . The administration of a pharmaceutical combination of the invention results in a beneficial effect, e.g. a synergistic or additive therapeutic effect, compared to monotherapy whereby only one of the pharmaceutically active ingredients is applied. Co-agents suitable for co-administration with the compound of the invention include but are not limited to:
(1) Interferons or conjugates of interferons such as:
   (a) Intron-A®, interferon alfa-2b (Schering Corporation, Kenilworth, NJ),
   (b) PEG-Intron®, peginteferon alfa-2b (Schering Corporation, Kenilworth, NJ),
   (c) Roferon®, recombinant interferon alfa-2a (Hoffmann-La Roche, Nutley, NJ),
   (d) Pegasys®, peginterferon alfa-2a (Hoffmann-La Roche, Nutley, NJ),
   (e) Berefor®, interferon alfa 2 available (Boehringer Ingelheim Pharmaceutical, Inc., Ridgefield, CT),
   (f) Sumiferon®, a purified blend of natural alpha interferons (Sumitomo, Japan),
   (g) Wellferon®, lymphoblastoid interferon alpha n1 (GlaxoSmithKline),
   (h) Infergen®, consensus alpha interferon (InterMune Pharmaceuticals, Inc., Brisbane, CA and Amgen, Inc., Newbury Park, CA),
   (i) Alferon®, a mixture of natural alpha interferons (Interferon Sciences, and Purdue Frederick Co., CT),
   (j) Viraferon®;
   (k) Conjugated interferons include, for example, Albuferon (Human Genome Science) which is conjugated to human albumin. Interferon conjugated to a water-soluble polymer or polyalkylene oxide homopolymers such as polyethylene glycol (PEG) or polypropylene glycols, polyoxyethylenated polyols, copolymers thereof and block copolymers thereof. As an alternative to polyalkylene oxide-based polymers, effectively non-antigenic materials such as dextran, polyvinyl pyrrolidones, polyacrylamides, polyvinyl alcohols, carbohydrate-based polymers and the like can be used. Interferon-polymer conjugates are described in US 4766106, US 4917888, EPA 0 236 987, EPA 0 510 356 and WO 95/13090. Since the polymeric modification sufficiently reduces antigenic responses, the foreign interferon need not be completely autologous. Interferon used to prepare polymer conjugates may be prepared from a mammalian extract, such as human, ruminant or bovine interferon, or recombinantly produced.

Other forms of interferons include interferon beta, gamma, tau and omega, such as Rebif ( Interferon beta 1a) by Serono, Omniferon (natural interferon) by Viragen, or Omega Interferon by Boehringer Ingelheim;

Oral interferons such as oral interferon alpha by Amarillo Biosciences;

In another aspect, additional examples of interferons include pegylated interferon alpha, for example pegylated interferon alpha -2a, pegylated interferon alpha -2b, pegylated consensus interferon or pegylated purified interferon- alpha product. Pegylated interferon alpha -2a is described in European Patent 593,868 and commercially available e. g. under the trade name PEGASYS^{®} (Hoffmann-La Roche). Pegylated interferon- alpha -2b is described, e.g. in European Patent 975,369 and commercially available e.g. under the trade name PEG-INTRON A^{®} (Schering Plough). Pegylated consensus interferon is described in WO 96/91953.

### (2) Antivirals

Antiviral agents may be compounds or biologicals that are effective to inhibit the formation and/or replication of a virus in a mammal. This includes agents that interfere with either host or viral mechanisms necessary for the formation and/or replication of a virus in a mammal such as ribavirin (1-beta-D-ribofuranosyl-1H-1,2,4-triazole-3-carboxamide) from Valeant Pharmaceuticals, Inc., Costa Mesa, CA, Rebetol® from Schering-Plough Corporation, Kenilworth, NJ, and Copegus® from Hoffmann-La Roche, Nutley, NJ, ribavirin analogues in development such as Levovirin and Viramidine by Valeant, and Mizoribine Monophosphate;

### (3) Protease inhibitors

Inhibitors of HCV NS3-4A serine protease such as substrate-based protease inhibitors (Attwood et al., *Antiviral peptide derivatives,* PCT WO 98/22496, 1998; Attwood et al., Antiviral Chemistry and Chemotherapy 1999, 10, 259-273; Attwood et al, Preparation and use of amino acid derivatives as anti-viral agents, German Patent Pub. DE 19914474; Tung et al. Inhibitors of serine proteases, particularly hepatitis C virus NS3 protease; PCT WO 98/17579), including alphaketoamides and hydrazinoureas, and inhibitors that terminate in an electrophile such as a boronic add or phosphonate (Llinas-Brunet et al. Hepatitis C inhibitor peptide analogues, PCT WO 99107734);

Protease inhibitors disclosed in U.S. patents for the treatment of HCV, for example, U.S. Patent No. 6,004,933 to Spruce et al which discloses a class of cysteine protease inhibitors for inhibiting HCV endopeptidase 2; U.S. Patent No. 5,990,276 to Zhang et al which discloses synthetic inhibitors of hepatitis C virus NS3 protease; U.S. Patent No. 5,538,865 to Reyes et al.; Peptides as NS3 serine protease inhibitors of HCV which are disclosed in WO 02/008251 to Corvas International, Inc., and WO 02108187 and WO 02/008256 to Schering Corporation HCV inhibitor tripeptides which are discloses in U.S. Patent Nos. 6,534,523, 6,410,531 and 6,420,380 to Boehringer Ingelheim and WO 02/060926 to Bristol Myers Squibb; Diaryl peptides as NS3 serine protease inhibitors of HCV which are disclosed in WO 02148172 to Schering Corporation; Imidazoleidinones as NS3 serine protease inhibitors of HCV which are disclosed in WO 02/18198 to Schering Corporation and WO 02/48157 to Bristol Myers Squibb; WO 98/17679 to Vertex Pharmaceuticals and WO 02148116 to Bristol Myers Squibb also disclose HCV protease inhibitors.
(4) Non-substrate-based NS3 protease inhibitors such as 2,4,6-trihydroxy-3-nitrobenzamide derivatives (Sudo K. et al., Biochemiscal and Biophysical Research Communications, 1997, 238 643-647; Sudo K. et al. Antiviral Chemistry and Chemotherapy, 1998, 9, 186), including RD3-4082 and RD3-4078;
(5) A phenanthrenequinone possessing activity against protease in a SDS-PAGE and autoradiography assay isolated from the fermentation culture broth of Streptomyces sp., Sch 68631 (Chu M. et al., Tetrahedron Letters, 1996, 37, 7229-7232),. (Chu M et al., Tetrahedron Letters 37:7229-7232, 1996); Sch 351633, isolated from the fungus *Penicillium grieofulvum* (Chu M. et al., Bioorganic and Medicinal Chemistry Letters 9:1949-1952); selective inhibitors designed based on the macromolecule eglin c., which is isolated from leech and is a potent inhibitor of several serine proteases such as S. griseus proteases A and B, V-chymotrypsin, chymase and subtilisin (Qasim M.A. et al., Biochemistry 36:1598-1607, 1997).
(6) Thiazolidines and benzanilides Identified in Kakiuchi N. et al. J. FEBS Letters 421, 217-220; Takeshita N. et al. Analytical Biochemistry, 1997, 247, 242-246. Thiazolidine derivatives which show relevant inhibition in a reverse-phase HPLC assay with an NS3/4A fusion protein and NS5A/5B substrate (Sudo K. et al., Antiviral Research, 1996, 32, 9-18). especially compound RD-16250 possessing a fused cinnamoyl moiety substituted with a long alkyl chain, RD4 6205 and RD4 6193
(7) HCV NS3-4A serine protease inhibitors including BILN 2061 by Boehringer Ingelheim, VX-950 by Vertex, SCH-503034 and SCH-351633, by Schering-Plough, and other HCV protease inhibitors in preclinical and clinical development by GlaxoSmithKline, Bristol Myers Squibb, Abbot, Roche, Merck, Pfizer, and Gilead;
(8) Nucleoside analogs

Telbivudine by Idenix (US 6444652, US6596700, and WO0196353). Nucleoside or non-nucleoside inhibitors of HCV NS5B RNA-dependent RNA polymerase, such as 2'-C-methyl-3'-O-L-valine ester ribofuranosyl cytidine (NM283, Idenix) as disclosed in WO 2004/002422.

Branched nucleosides disclosed by Idenix Pharmaceuticals for the use of treatment of flaviviruses (including HCV) and pestiviruses in International Publication Nos. WO 01/90121 and WO 01/92282. Specifically, a method for the treatment of hepatitis C infection (and flaviviruses and pestiviruses) in humans and other host animals is disclosed in the Idenix publications that includes administering an effective amount of a biologically active 1', 2', 3' or 4'-branced B-D or B-L nucleosides or a pharmaceutically acceptable salt or prodrug thereof, administered either alone or in combination with another antiviral agent, optionally in a pharmaceutically acceptable carrier.

Nucleoside analogs in other patent applications disclosing the use of treatment of hepatitis C virus include: PCTCA00/01316 (WO 01/32153; filed November 3, 2000) and PCT/CA01/00197 (WO 01/60315; filed February 19, 2001) filed by BioChem Pharma, Inc., (now Shire Biochem, Inc.); PCT/US02/01531 (WO 02/057425; filed January 18, 2002) and PCT/US02/03086 (WO 02/057287; filed January 18, 2002) filed by Merck & Co., Inc., PCT/EP01/09633 (WO 02/18404; published August 21, 2001) filed by Roche, and PCT Publication Nos. WO 01/79246 (filed April 13, 2001), WO 02/32920 (filed October 18, 2001) and WO 02/48165 by Pharmasset, Ltd.
2'-fluoronucleosides disclosed in PCT Publication No. WO 99/43691 to Emory University, entitled "2'-Fluoronucleosides";
2'-modified nucleosides disclosed by Eldrup et al. (Oral Session V, Hepatitis C Virus, Flaviviridae; 16th International Conference on Antiviral Research (April 27, 2003, Savannah, GA));
Nucleoside analogues and 2'-modified nucleosides disclosed in Bhat et al. (Oral Session V, Hepatitis C Virus, Flaviviridae, 2003 (Oral Session V, Hepatitis C Virus, Flaviviridae; 16th International conference on Antiviral Research (April 27, 2003, Savannah, Ga); p A75);
2'-modified nucleosides disclosed in Olsen et al. (Oral Session V, Hepatitis C Virus, Flaviviridae; 16th International Conference on Antiviral Research (April 27, 2003, Savannah, Ga)p A76)
Preclinical compounds include R803 (Rigel), JTK-003 (Japan Tabacco), HCV-086 (ViroPharma/Wyeth), R-1479 (Roche);
(9) Nucleotide polymerase inhibitors and gliotoxin (Ferrari R. et al. Journal of Virology, 1999, 73, 1649-1654), and the natural product cerulenin (Lohmann V. et al. Virology, 1998, 249, 108-118);
(10) HCV NS3 helicase inhibitors, such as VP_50406 by ViroPhama and compounds from Vertex, other helicase inhibitors disclosed in U.S. Patent No. 5,633,388 and PCT WO 97/36554;
(11) Antisense molecules directed against HCV genome or any cellular component that is required for viral replication.
   Phosphorothioate oligodeoxynucleotides (S-ODN) complementary to sequence stretches in the 5' non-coding region (NCR) of the virus (Alt M. et al., Hepatology, 1995, 22, 707-717), or nucleotides 326-348 comprising the 3' end of the NCR and nucleotides 371-388 located in the core coding region of the HCV RNA (Alt M. et al., Archives of Virology, 1997, 142, 589-599; Galderisi U. et al., Journal of Cellular Physiology, 199, 181, 251-257); such as ISIS 14803 by Isis Pharm/Elan, antisense oligonucleotides by Hybridon, AVI bioPharma and Chugai, AVI-4065 (AVI BioPharma), or an antisense sequence complementary to any part of the HCV genome which increases effectiveness of therapy;
(12) Inhibitors of IRES-dependent translation (Ikeda N et al., *Agent for the prevention and treatment of hepatitis C,* Japanese Patent Pub. JP-08268890; Kai Y et al. *Prevention and treatment of viral diseases,* Japanese Patent Pub. JP-10101591); such as ISIS 14803 by Isis Pharm/Elan, IRES inhibitors by PTC Therapeutics, Anadys, Immusol, RiboTargets, and SomaGenics;
(13) Ribozymes, such as nuclease-resistant ribozymes (Maccjak, D.J. et al., *Hepatology* 1999, 30, abstract 995) and those directed in U.S. Patent No. 6,043,077 to Barber et al., and U.S. Patent Nos. 5,869,253 and 5,610,054 to Draper et al. for example, HEPTAZYME by RPI, and Sirna Therapeutics Inc.;
(14) An inhibitor of other targets in the HCV life cycle including viral entry, assembly and maturation such as Celgosivir (MBI 3253), a glycoprotein processing inhibitor by Migenix, fusion inhibitor by Trimeris, ACH-0137171 by Achillion;
(15) Immune modulating agents.
   Receptor agonists such as toll like receptor (;TLR') agonists include ANA245, ANA971, ANA975 (US 5041426, 4880784) by Anadys; CpG-10101 a TLR-9 agonist (Coley Pharmaceuticals); an IMPDH inhibitor, mycophenolic acid, a salt or a prodrug thereof sodium mycophenolate or mycophenolate mofetil, or Merimebodib (VX-497, by Vertex); thymosin alpha-1 (Zadaxin or its combination, by SciClone); SCV-07 (SciClone), Belerofon (improved IFN-α by Nautilus); CIVACIR (hepatitis C Immune Globulin) by NABI, or a S1P receptor agonist, e.g. FTY720 or analogue thereof optionally phosphorylated, e.g. as disclosed in EP627406A1, EP778263A1, EP1002792A1, WO02/18395 WO02/76995, WO 02/06268, JP2002316985, WO03/29184, WO03/29205, WO03/62252 and WO03/62248 ; Resiquimod [VML 600] by 3M Pharmaceuticals, an imiquimod analogue that is a potent inducer of interferon-α and other cytokines;
(16) An anti-fibrotic agent, such as a N-phenyl-2-pyrimidine-amine derivative, imatinib (Gleevac), IP-501 by Indevus, and Interferon gamma 1b from InterMune, Pirfenidone (Multiple: TGF Beta agonist, FGF antagonist, PDGF antagonist) by Intermune, Marnac, Shionogi;
(17) Therapeutic vaccines by Intercell (Therapeutic peptide vaccine IC41 HCV), Epimmune/Genecor, Merix, Tripep (Chiron-VacC), immunotherapy (Therapore) by Avant, T cell therapy by CellExSys, monoclonal antibody XTL-002 by STL, ANA 246 and ANA 246 by Anadys, a therapeutic vaccine directed to E2 by Innogenetics, mAb against E2 envelope protein by XTL Bio, GI-5005 (Globelmmune Inc), InnoVac-C (WO 9967285, Innogenetics), IC-41 (Intercell), interferon alfa-n3 (Interferon Sciences), Engerix B (SmithKline Beecham);
(18) Other compounds include Ursodiol (EP00269516, Axcan Pharma), HE-2000 (a DHEA analog, Colthurst Ltd), EHC-18 (an immunomodulator, Enzo biochem), histamine dihydrochloride (an H2 agonist, WO09104037, Estero-Anstalt), 1-amino-alkylcyclohexanes (U.S. Patent No. 6,034,134), alkyl lipids (U.S. Pat. No. 5,922,757), vitamin E and other antitoxidants (U.S. Patent. No. 5,922,757), bile acids (U.S. Pat. No. 5,846,99964), N-(phosphonoacetl)-L-aspartic acid) U.S. Pat. No. 5,830,905), benzenedicarboxamides (U.S. Pat. No. 5,633,388), polyadenylic acid derivatives (U.S. Pat. No. 5,496,546), 2'3'-dideoxyinosine (U.S. Pat. No. 5,026,687), benzimidazoles (U.S. Pat. No. 5,891,874), plant extracts (U.S. Pat. No. 5,837,257, U.S. Pat. No. 5,725,859 and U.S. Pat. No. 6,056,961) and piperidines (U.S. Pat. No. 5,830,905); N-(phosphonoacetyl)-L-aspartic acid, benzenedicarboxamides, polyadenylic acid derivatives, glycosylation inhibitors, and nonspecific cytoprotective agents that block cell injury caused by the virus infection;
(19) Any other compound currently in preclinical or clinical development for the treatment of HCV, including Interleukin-10 (Schering-Plough), AMANTADINE (Symmetrel) by Endo Labs Solvay, caspase inhibitor IDN-6556 by Idun Pharma, HCV/MF59 by Chiron, CEPLENE (histamine dichloride) by Maxim, IDN-6556 by Idun PHARM, T67, a beta-tubulin inhibitor by Tularik, FK788 by Fujisawa Healthcare, IdB1016 (Siliphos, oral silybin-phosphatidyl choline phytosome), Dication by Immtech, hemopurifier by Aethlon Medical, UT 231 B by United Therapeutics; HepeX-C SM1, PPVO-Bay55-8800 (Parapoxvirus ovis) by Bayer; Refanalin(HGF mimetic) by Angion, R803 (Rigel), JTK-003, JTK-002, and JTK-1 09 (all from Japan Tobacco), HCV-086 (ViroPharma/Wyeth), ISIS-14803 (ISIS Pharmaceuticals), GS-9132 (a polymerase inhibitor by Achillion Pharmaceuticals), HCV-793 (Pharmasset and Roche), R1626 (Roche).
   Compounds or drugs in preclinical development can easily be identified by one of skill in the art by searching clinical trial information on the internet, for example, and press release information from respective companies
(20) Compounds which increase effectiveness of combination therapy including an antifolate, a 5-fluoropyrimidine (including 5-fluorouracil), a cytidine analogue such as β-L-1,3-dioxolanyl cytidine or β-L-1,3-dioxolanyl 5-fluorocytidine, antimetabolites (including purine antimetabolites, cytarabine, fudarabine, floxuridine, 6-mercaptopurine, methotrexate, and 6-thioguanine), hydroxyurea, mitotic inhibitors (including CPT-11, Etoposide (VP-21), taxol, and vinca alkaloids such as vincristine and vinblastine, an alkylating agent (including but not limited to busulfan, chlorambucil, cyclophosphamide, ifofamide, mechlorethamine, melphalan, and thiotepa), nonclassical alkylating agents, platinum containing compounds, bleomycin, an antitumor antibiotic, an anthracycline such as doxorubicin and dannomycin, an anthracenedione, topoisomerase II inhibitors, hormonal agents (including but not limited to corticosteroids (dexamethasone, prednisone, and methylprednisone), androgens such as fluoxymesterone and methyltestosterone, estrogens such as diethylstilbesterol, antiestrogens such as tamoxifen, LHRH analogues such as leuprolide, antiandrogens such as flutamide, aminoglutethimide, megestrol acetate, and medroxyprogesterone), asparaginase, carmustine, lomustine, hexamethyl-melamine, dacarbazine, mitotane, streptozocin, cisplatin, carboplatin, levamasole, and leucovorin. The compounds of the present invention can also be used in combination with enzyme therapy agents and immune system modulators such as interleukin, tumor necrosis factor, macrophage colony-stimulating factor and colony stimulating factor.

Utility of the a cyclophilin-binding compound or therapeutic agent such as sanglifehrin in treating diseases and conditions as hereinabove specified may be demonstrated in standard in vitro or clinical tests, e.g. in accordance with the methods described hereinafter.

### A. In vitro

The effects of compounds of formula XVI on the replication of the HCV genome are examined using the HCV replicon cells.

The HCV replicon cell line, clone A, is licensed from Apath, LLC. The cells are cultured in Dulbecco's modified Eagle's medium (DMEM, Gibco), containing 10% heat-inactivated fetal bovine serum (FBS, Gibco), 2 mM L-glutamine, 1x nonessential amino acids (Gibco), and 1 mg/ml G418 (Invitrogen, Carlsbad, CA).

To determine antiviral effect of the compounds, HCV replicon cells (clone A) are treated with compounds serially diluted in DMEM supplemented with 2% FBS and 0.5% DMSO for 48 h, then the total intracellular RNA was extracted, and the level of HCV RNA is determined by a real-time quantitative RT-PCR (Taqman) using HCV-specific primers (5'-TCT TCA CGC AGA AAG CGT CTA-3' and 5'-CTG GCA ATT CCG GTG TAC T-3) Sequence ID No: 1 and probe (5'-6-FAM-TCC TGG AGG CTG CAC GAC ACT CAT A-TAMRA-3') Sequence ID No: 2. For each treatment, the quantity of HCV RNA is normalized against the amount of total RNA extracted, which is determined in a Quant-iT assay (Molecular Probe). Each data point obtained represents the average of six replicates in cell culture. IC₅₀ is the concentration of the compound at which the HCV RNA level in the replicon cells is reduced by 50%. To monitor cytotoxic effect of the compounds, the viability of the replicon cells following 48 h of compound treatment is determined using a tetrazolium compound (MTS)-based assay (CellTiter 96® AQueous One Solution Cell Proliferation Assay, Promega, Madison, WI). CC₅₀ is the concentration of the compound at which the cell viability is reduced by 50%.

### B. Clinical Trial

A total of 15∼30 patients with hepatitis C viral infection are enrolled in a study of 2∼12 weeks. Each patient receives a therapeutic effective dose of a cyclophilin-binding compound of formula XVI. The serum levels of viral RNA load are monitor over the course of treatment and in a follow-up period of 2 weeks typically for all patients.

The length of treatment and dosages required to achieve sustained virological responses in hepatitis C patients are to be determined in further clinical trials involving greater number of patients and longer treatment time of up to 12 months with 6 months follow-up.

Daily dosages required will vary depending upon, for example, the cyclophilin-binding compound employed, the host, the mode of administration, the severity of the condition to be treated.

The compound of formula XVI may be administered as the sole ingredient or together with other drugs, e.g. a drug which has anti-HCV activities, e.g. an interferon alpha, a pegylated interferon alpha, ribavirin, an HCV polymerase inhibitor such as NM283, or an HCV protease inhibitor such as SCH503034 and VX-950. Daily dosages with respect to the co-agents used will vary depending upon, for example, synergistic effects with the compound employed, the host, the mode of administration and the severity of the condition to be treated.

## Claims

1. A cyclophilin-binding compound for use in the treatment of hepatitis C, liver fibrosis, liver cirrhosis and hepatocellular carcinoma,
wherein the cyclophilin-binding compound is a compound of the formula 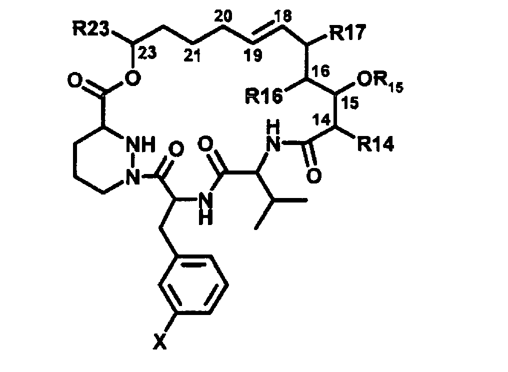 wherein X is H, methoxy, ethoxy, OnPr, OiPr,
R14 and R15 are independently H, methyl, alkyl, (C₁-C₄)alkyl, (C₁-C₄)-CO-CH₃, or R14 and R15 are fused to form a 5 to 7 membered substituted or unsubstituted cycloalkyl: selected of formula 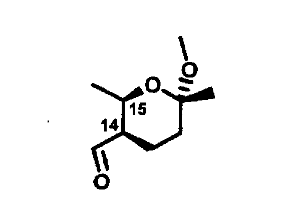 R16 is H, or Me or (C₁₋₄)alkyl;
R17 is H, OH, -O-(C₁₋₄)alkyl or R14, R15 and R17 are fused to form a cycloalkyl of formula 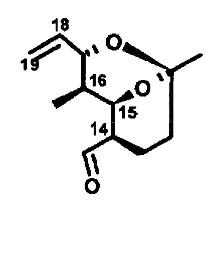 R23 is selected from the group consisting of -CH=CH₂,-CH₂-OH, -CH₂-OCH₃, -CHO, - COOH,

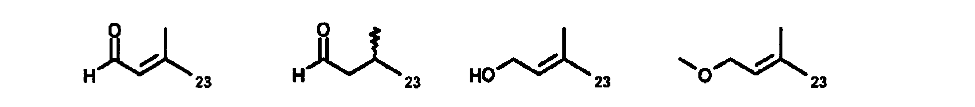

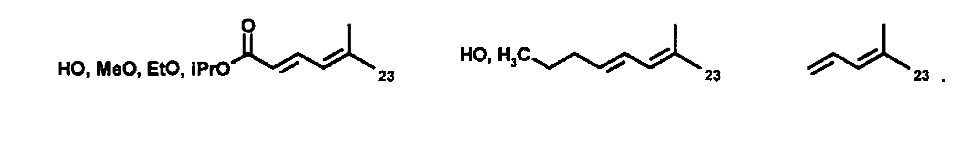

## Patentansprüche

1. Cyclophilin bindende Verbindung zur Verwendung bei der Behandlung von Hepatitis C, Leberfibrose, Leberzirrhose und hepatozellulärem Karzinom,
wobei es sich bei der Cyclophilin bindenden Verbindung um eine Verbindung der Formel 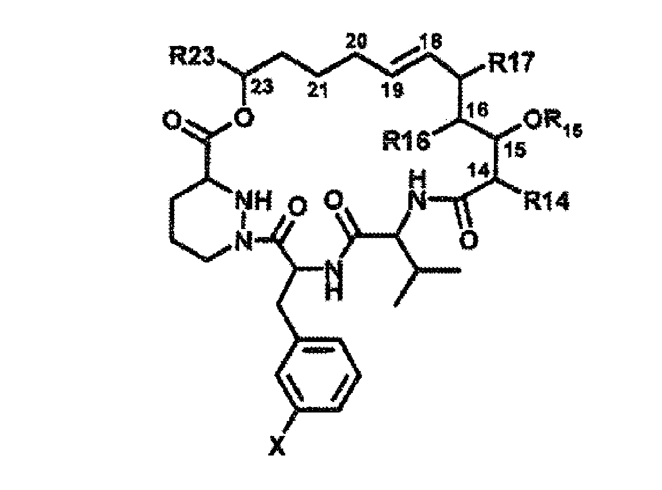 handelt, wobei X für H, Methoxy, Ethoxy, OnPr, OiPr steht,
R14 und R15 unabhängig für H, Methyl, Alkyl, (C₁-C₄)Alkyl, (C₁-C₄)-CO-CH₃ stehen oder R14 und R15 unter Bildung eines 5- bis 7-gliedrigen gegebenenfalls substituierten Cycloalkyls: ausgewählt aus der Formel 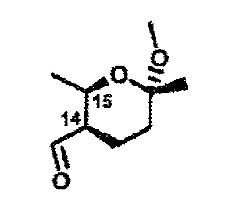 kondensiert sind,
R16 für H oder Me oder (C₁₋₄)Alkyl steht,
R17 für H, OH, -O-(C₁₋₄)Alkyl steht oder R14, R15 und R17 unter Bildung eines Cycloalkyls der Formel 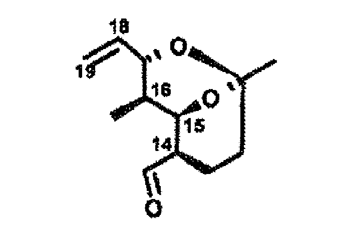 kondensiert sind,
R23 ausgewählt ist aus der Gruppe bestehend aus -CH=CH₂, -CH₂-OH, -CH₂-OCH₃, -CHO, -COOH,

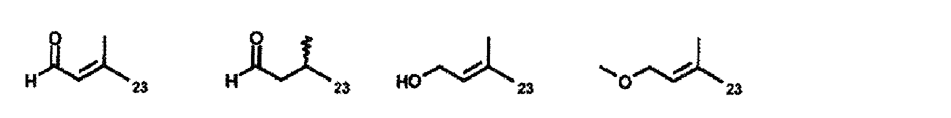

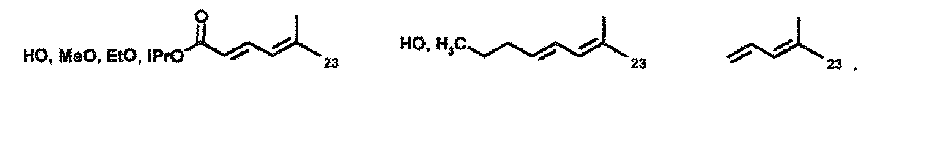

## Revendications

1. Composé de liaison de cyclophiline pour utilisation dans le traitement de l'hépatite C, la fibrose hépatique, la cirrhose du foie et le carcinome hépatocellulaire,
où le composé de liaison de cyclophiline est un composé de formule 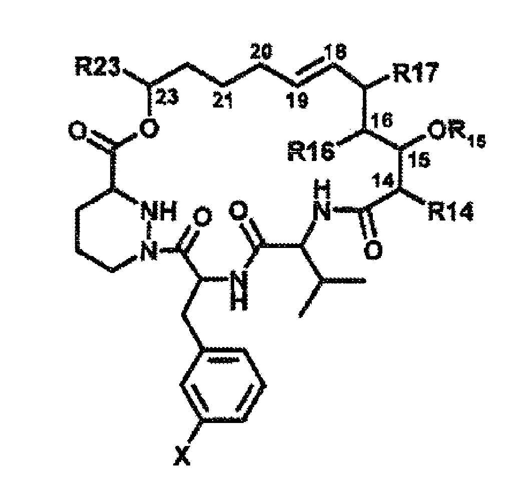 dans laquelle X est H, un méthoxy, un éthoxy, OnPr, OiPr ;
R14 et R15 sont indépendamment H, un méthyle, un alkyle, un alkyle en C₁-C₄, (C₁-C₄)-CO-CH₃, ou R14 et R15 sont condensés pour former un cycloalkyle de 5 à 7 chaînons substitué ou non substitué choisi de formule 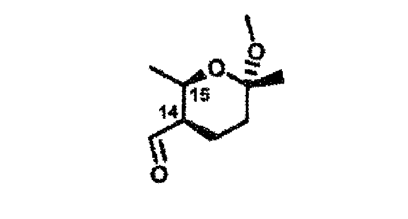 R16 est H, ou Me ou un alkyle en C₁-C₄ ;
R17 est H, OH, -O-(alkyle en C₁-C₄) ou R14, R15 et R17 sont condensés pour former un cycloalkyle de formule 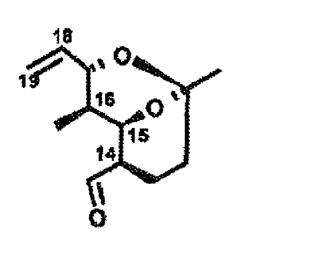 R23 est choisi dans le groupe constitué de -CH=CH₂, -CH₂-OH, -CH₂-OCH₃, -CHO, -COOH,
